# NOUVEAU FASCICULE DE BREVET EUROPEEN

(11) **EP 0 401 130 B2**
(45) Date de publication et mention de la décision concernant l'opposition: **26.05.1999**
(45) Mention de la délivrance du brevet: 24.11.1993
(21) Numéro de dépôt: 90420229.8
(22) Date de dépôt: 16.05.1990
(51) Int. Cl.: A61M 1/14

(54) **Dispositif de préparation de solutions à usage médical**
Einrichtung zur Vorbereitung medizinischer Lösungen
Device for the preparation of medical solutions

(30) Priorité: 29.05.1989 FR 8907272; 29.05.1989 FR 8907273; 29.05.1989 FR 8907274
(43) Date de publication de la demande: 05.12.1990
(73) Titulaire: HOSPAL INDUSTRIE, F-69883 Meyzieu Cédex (FR)
(72) Inventeur: Gauckler, Jacques, F-69005 Lyon (FR); Chevallet, Jacques, F-69360 Serezin du Rhone (FR); Burtin, Jacques, F-69320 Feyzin (FR)
(74) Mandataire: Lejeune, Daniel

(56) Documents cités:
- EP-A- 0 278 100
- FR-A- 2 569 560
- GB-A- 2 025 787
- US-A- 4 621 928

## Description

La présente invention concerne un dispositif pour la préparation de solutions à usage médical par mélange d'un liquide avec au moins un sel pulvérulent ou cristallisé. Ces solutions sont utilisables notamment en hémodialyse, en hémofiltration ou en hémodiafiltration. Un tel dispositif permet la préparation en ligne du liquide de dialyse nécessaire en hémodialyse ou en hémodiafiltration ou des liquides de substitution à l'ultrafiltrat de sang prélevé au patient, nécessaires en hémofiltration ou en hémodiafiltration.

Par la demande de brevet européen 278 100, il est connu un dispositif de préparation d'une solution à usage médical à partir d'eau et d'au moins un sel pulvérulent comprenant notamment :
- une canalisation principale ayant une première extrémité reliée à une source d'eau et une seconde extrémité pour délivrer la solution ;
- au moins une canalisation secondaire reliée en dérivation à la canalisation principale, cette canalisation secondaire comprenant un réservoir contenant un sel pulvérulent et ayant un orifice d'entrée et un orifice de sortie situés de préférence respectivement à un point haut et à un point bas du réservoir ;
- des moyens pour faire circuler du liquide dans les canalisations.

L'ensemble des canalisations est à la pression atmosphérique.

Avec cette disposition, on a remarqué que l'eau, qui circule donc de haut en bas dans le réservoir, a tendance à créer des chemins préférentiels dans le sel pulvérulent et que le sel, en s'humidifiant, forme localement des agglomérats relativement difficiles à dissoudre, Ces deux phénomènes - la création de chemins préférentiels et la formation d'agglomérats - sont indésirables en ce qu'ils s'opposent à la dissolution régulière et complète du sel, qui est nécessaire au bon déroulement d'une préparation en ligne de solution.

La présente invention a donc pour but un dispositif de préparation de solution à usage médical qui permette d'obtenir une dissolution régulière du sel et ce, quel que soit son coefficient de solubilité.

Pour atteindre ce but on prévoit, conformément à' l'invention, un premier dispositif de préparation d'une solution à usage médical à partir d'un liquide et d'au moins un sel pulvérulent ou cristallisé, comprenant :
- une canalisation principale ayant une première extrémité reliée à une source de liquide et une seconde extrémité pour délivrer la solution;
- au moins une canalisation secondaire reliée en dérivation à la canalisation principale, cette canalisation secondaire comprenant un réservoir contenant un sel pulvérulent ou cristallisé et ayant un orifice d'entrée et un orifice de sortie situés de préférence respectivement à un point haut et à un point bas du réservoir,
- des moyens pour faire circuler du liquide dans les canalisations comprenant
- des moyens de pompage disposés en aval du réservoir sur la canalisation secondaire,
   ce dispositif étant caractérisé en ce qu'il comprend des moyens pour provoquer l'immersion du sel dans le reservoir en fermant initialement des moyens d'obturation disposés sur la canalisation secondaire en amont du réservoir en mettant en fonctionnement les moyens de pompage et en ouvrant subséquemment les moyens d'obturation.

Grâce à cette disposition, il est possible, avant de procéder au remplissage initial des canalisations, de vider le réservoir de l'air qu'il contient, de sorte que le réservoir se remplit totalement d'eau lors du remplissage initial et reste rempli par la suite.

Conformément à l'invention, un second dispositif de préparation d'une solution à usage médical à partir d'un liquide et d'au moins un sel pulvérulent ou cristallisé, comprend :
- une canalisation principale ayant une première extrémité reliée à une source de liquide et une seconde extrémité pour délivrer la solution;
- au moins une canalisation secondaire reliée en dérivation à la canalisation principale, cette canalisation secondaire comprenant un réservoir contenant un sel pulvérulent ou cristallisé et ayant un orifice d'entrée et un orifice de sortie situés de préférence respectivement à un point haut et à un point bas du réservoir,
- des moyens pour faire circuler du liquide dans les canalisations,
   ce dispositif étant caractérisé en ce qu'il comprend des moyens pour provoquer l'immersion du sel dans le réservoir comprenant des moyens de mise en pression du réservoir.

Grâce à cette disposition, la hauteur d'eau dans le réservoir peut être ajustée par réglage de la pression de l'air dans le réservoir de façon que le niveau de l'eau s'établisse, lors du remplissage initial, au-dessus de la surface supérieure du sel pulvérulent.

Avec ces deux dispositifs, le sel reste à l'état fractionné en permanence et la solution qui est soutirée du réservoir présente une concentration sensiblement constante, qui est celle de la saturation pour les sels généralement utilisés pour la préparation de solutions à usage médical, le chlorure de sodium et le bicarbonate de sodium notamment.

Selon une caractéristique de l'invention, la canalisation secondaire comporte une boucle comprenant le réservoir et des moyens pour faire circuler du liquide dans la bouche en circuit fermé.

Grâce à cette disposition, le débit du liquide dans la boucle est indépendant du débit dans le reste du dispositif et ce débit peut être réglé pour que, à tout moment, quel que soit le coefficient de solubilité du sel, la solution circulant dans la boucle soit saturée.

Selon une autre caractéristique de l'invention, le point de jonction de la canalisation principale à la portion de canalisation secondaire qui est reliée à l'orifice d'entrée du réservoir est situé en aval, par rapport au sens de circulation du liquide dans la canalisation principale, du point de jonction de la canalisation principale à la canalisation secondaire qui est reliée à l'orifice de sortie du réservoir.

Cette disposition est particulièrement avantageuse dans le cas de sels ayant un faible coefficient de solubilité puisque, en permettant une recirculation dans la canalisation secondaire, elle a pour conséquence l'élévation de la concentration du liquide circulant dans la canalisation secondaire.

D'autres caractéristiques et avantages de la présente invention apparaîtront à la lecture de la description qui va suivre, faite en relation aux dessins annexés sur lesquels:
La figure 1 représente, de façon schématique, un premier mode de réalisation du dispositif selon l'invention;
La figure 2 représente, de façon schématique, un deuxième mode de réalisation du dispositif selon l'invention;
La figure 3 représente, de façon schématique, un troisième mode de réalisation du dispositif selon l'invention; et
La figure 4 représente, de façon schématique, un quatrième mode de réalisation du dispositif selon l'invention.

Dans la suite, les éléments homologues des différents modes de réalisation du dispositif selon l'invention sont désignés par les mêmes chiffres de référence. En outre, pour plus de clarté, on n'a représenté sur les figures que les éléments essentiels à la compréhension de la présente invention, en négligeant volontairement tous les accessoires utilisés en pratique, tels que les dispositifs de chauffage, de dégazage, de purge, de nettoyage, de stérilisation, de prises d'échantillons, etc, qui sont classiques.

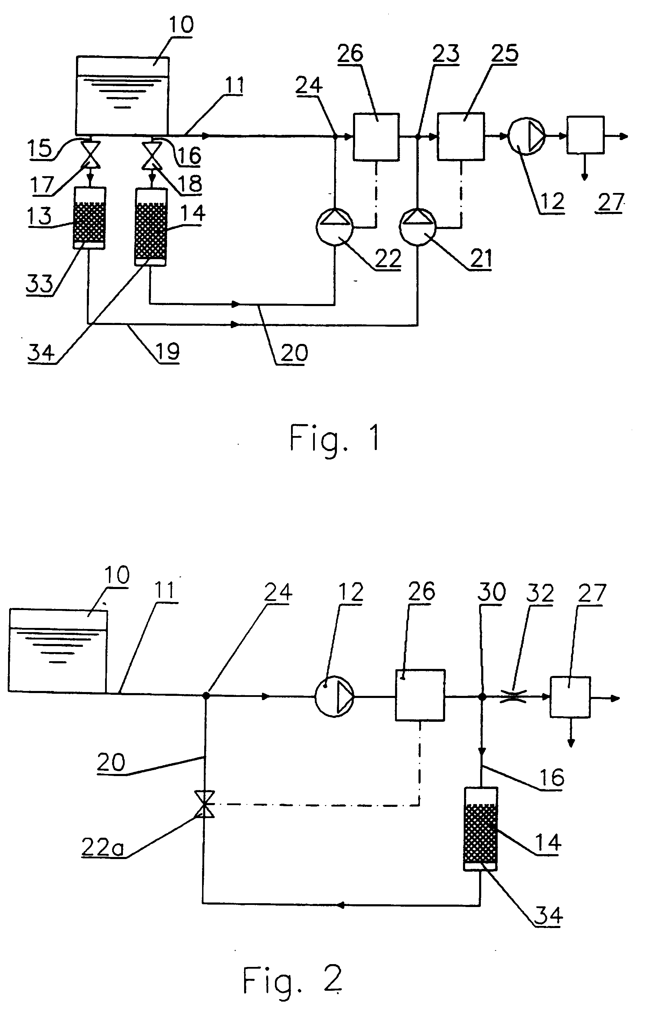

Le dispositif représenté sur la figure 1 comporte une canalisation principale 11 ayant une extrémité reliée à une source 10 de liquide, qui peut être de l'eau ou une solution, et dont l'autre extrémité est reliés à une unité de distribution en ligne et de contrôle 27 de la solution à usage médical. Une pompe 12, disposée sur la canalisation principale, assure la circulation de liquide dans cette canalisation, de la source de liquide 10 vers l'unité de distribution 27. Deux canalisations secondaires, de structure analogue, sont reliées en parallèle à la canalisation principale 11, chacune de ces canalisations secondaires formant une dérivation à la canalisation principale.

Chaque canalisation secondaire comprend un réservoir 13 (14) contenant un type de sel pulvérulent (bicarbonate de sodium, chlorure de sodium, par exemple), ce réservoir ayant un orifice d'entrée relié par un conduit 15 (16) à la source de liquide 10 et un orifice de sortie relié par un conduit 19 (20) à la canalisation principale 11, en un point de jonction 23 (24). Un organe d'obturation, tel qu'une vanne 17 (18), est disposé sur le conduit 15 (16), c'est-à-dire en amont du réservoir 13 (14) par rapport au sens de circulation du liquide dans la canalisation secondaire, et une pompe 21 (22) est disposés sur le conduit 19 (20), c'est-à-dire en aval du réservoir 13 (14). En fonctionnement, la pompe 21 (22) fait circuler le liquide dans la canalisation secondaire, du réservoir 10 vers le point de jonction 23 (24).

Les orifices d'entrée et de sortie des réservoirs 13, 14 sont respectivement situés à un point haut et à un point bas de ces réservoirs. Une grille 33 (34) disposée dans la partie basse de chaque réservoir 13 (14) empêche le sel non dissous de s'écouler par le conduit 19 (20).

Deux dispositifs de mesure 25, 26 de la concentration en sel des liquides s'écoulant dans la canalisation principale 11 sont disposés sur la canalisation 11 respectivement en aval des points de jonction 23, 24. Ces dispositifs de mesure 25, 26 pilotent les pompes 21, 22 en fonction de valeurs de consigne fixées par le clinicien tandis que l'unité de distribution en ligne et de contrôle 27 dévie la solution produite vers l'égoût tant que cette solution ne présente pas la concentration requise dans les différents sels.

La mise en oeuvre du dispositif qui vient d'être décrit est la suivante :le remplissage initial (priming) des canalisations primaire et secondaire par le liquide contenu dans le réservoir 10 est précédée d'une étape de mise sous vide partiel des réservoirs 13, 14. Pour ce faire, les canalisations secondaires sont obturéss en amont des réservoirs 13, 14 au moyen des vannes 17, 18 et les pompes 21, 22 sont mises en fonctionnement de façon à mettre les réservoirs en dépression par rapport à la source de liquide 10 (où règne, par exemple, une pression égale ou supérieure à la pression atmosphérique). Lorsque le niveau de vide requis est atteint, les vannes 17 et 18 sont ouvertes et les canalisations secondaires se remplissent totalement de liquide, de sorte que les sels pulvérulents contenus dans les réservoirs 13, 14 sont complètement immergés et le resteront jusqu'à dissolution totale.

La figure 2 représente un deuxième mode de réalisation du dispositif selon l'invention comprenant une canalisation principale 11 et une canalisation secondaire formant une dérivation à la canalisation principale. La canalisation principale 11, comme précédemment, a ses extrémités reliées respectivement à une source de liquide 10 (eau ou solution) et à une unité de distribution en ligne et de vérification 27 de la solution à usage médical.

La canalisation secondaire comprend un réservoir 14 de sel pulvérulent ayant un orifice d'entrée relié par un conduit 16 à la canalisation principale 11, en un point de jonction 30, et un orifice de sortie relié par un conduit 20 à la canalisation principale 11, en un point de jonction 24. Le point de jonction 30 est situé en aval du point de jonction 24 par rapport au sens de circulation du liquide dans la canalisation 11. Les orifices d'entrée et de sortie du réservoir 14 sont situés respectivement à un point haut et à un point bas du réservoir.

La circulation des liquides dans les canalisations principale et secondaire est provoquée par une pompe 12 disposée sur la canalisation 11 entre les points de jonction 24 et 30. Le sens de circulation du liquide dans la canalisation secondaire, selon lequel le liquide recircule partiellement dans les canalisations, est imposé par une perte de charge située sur la canalisation principale 11 en aval du point de jonction 10. Cette perte de charge peut être provoquée par un organe non spéciquement prévu à cette fin, l'unité de distribution et de contrôle 27, par exemple. Elle peut être également provoquée par des moyens spécifiques, comme l'organe d'étranglement 12. C'est cette perte de charge qui permet aussi la mise en pression du réservoir 14 sous l'action de la pompe 12, et donc le remplissage du réservoir 14 au niveau souhaité.

Comme le dispositif précédent, ce dispositif comporte un organe de mesure 26 de la concentration en sel de la solution circulent dans la canalisation principale. Cet organe est disposé en aval du point de jonction 24 et il pilote une vanne 22a disposée sur la canalisation secondaire en aval du réservoir 14 de façon que la solution circulant dans la canalisation principale ait la concentration en sel souhaitée.

La mise en oeuvre de ce dispositif de préparation d'une solution à usage médical est la suivante : le remplissage initial des canalisations principale et secondaire est provoqué par la mise en fonctionnement de la pompe 12 qui fait circuler le liquide provenant de la source 10 et met en pression une partie des canalisations, dont le réservoir 14. Un réglage judicieux de la perte de charge en aval du point de jonction 30 au moyen de l'organe d'étranglement 32 permet une mise en pression du réservoir suffisante pour garantir l'immersion du sel contenu dans le réservoir 14 dès la mise en marche du dispositif. L'unité de distribution en ligne et de contrôle 27 dévie la solution produite vers l'égoût tant que cette solution ne présente pas la concentration en sel requise.

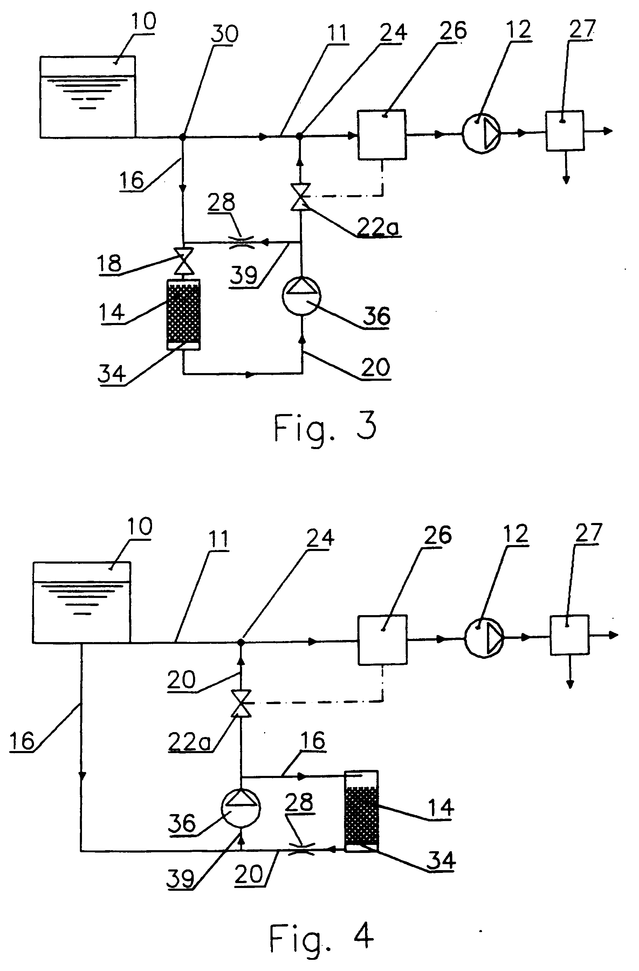

Les figures 3 et 4 représentent deux variantes du dispositif selon l'invention ayant pour principal point commun que leur canalisation secondaire comporte une boucle comprenant le réservoir de sel pulvérulent et des moyens pour faire circuler le liquide dans la boucle en circuit fermé. Dans le dispositif représenté sur la figure 3, la boucle est fermée par un conduit 39 qui relie le conduit 16 au conduit 20 (lesquels, comme précédemment, relient respectivement les orifices d'entrée et de sortie du réservoir 14 à la canalisation principale 11). Dans la boucle ainsi formée sont disposés une vanne 18 sur le conduit 16, c'est-à-dire en amont du réservoir 14, une pompe 36 sur le conduit 20, c'est-à-dire en aval du réservoir 14, et un organe d'étranglement 28 sur le conduit 39. L'organe de mesure 26 disposé sur la canalisation principale 11 en aval du point de jonction 24 du conduit 20 à la canalisation principale pilote une vanne 22a disposée sur le conduit 20, en aval de la boucle.

Le dispositif qui vient d'être décrit fonctionne de la façon suivante : avant d'effectuer le remplissage initial des canalisations principale et secondaire, la vanne 18 est fermée, la vanne 22a est ouverte et la pompe 36 est mise en fonctionnement de façon à créer un vide partiel dans le réservoir 14. Le niveau de vide désiré étant atteint, la vanne 18 est ouverte. Lorsque les canalisations sont complètement remplies, la vanne 22a est fermée et le liquide circule alors en circuit fermé dans la boucle sous l'action de la pompe 36 dont le débit est ajusté pour que le liquide dans la boucie soit rapidement saturé. La vanne 22a est alors ouverte et une partie de la solution saturée contenue dans la boucle s'écoule dans la canalisation principale 11 où elle se mélange avec le liquide aspiré de la source 10 par la pompe 12, tandis qu'elle est simultanément remplacée dans la boucle pardu liquide aspiré de la source 10, via le conduit 16, par la pompe 36. L'organe d'étranglement 28 disposé sur le conduit 39 a pour fonction de favoriser l'entrée de liquide et la sortie de solution dans/hors de la boucle lorsque la vanne 22a est ouverte. Comme dans les dispositifs décrits précédemment, l'unité de distribution en ligne et de contrôle 27 dévie la solution préparée vers l'égoût tant que sa concentration en sel n'a pas atteint le niveau requis.

Le dispositif représenté sur la figure 4 se différencie de celui qui vient d'être décrit en ce que la canalisation secondaire est fermée pour former une boucle par un conduit 39 qui est une portion de conduit commun aux conduits 16 et 20, sur laquelle est disposée une pompe 36 qui aspire le liquide provenant de la source 10 et la solution sortant du réservoir 14 et refoule en direction de la canalisation principale 11 et de l'entrée du réservoir 14. La boucie comprend en outre un organe d'étranglement 28 disposé en aval du réservoir 14 sur le conduit 20. L'organe de mesure 26 disposé sur la canalisation principale 11 en aval du point de jonction 24 du conduit 20 à la canalisation principale pilote une vanne 22a disposée sur le conduit 20, en aval de la boucle.

La mise en oeuvre du dispositif qui vient d'être décrit est la suivante : pour le remplissage initial des canalisations, la vanne 22a est fermée et les pompes 36 et 12 sont mises en fonctionnement. La pompe 36 remplit la canalisation secondaire avec le liquide de la source 10 tout en mettant en pression le réservoir 14 grâce à la perte de charge provoquée par l'organe d'étranglement 28. Par suite de cette mise en pression, qui peut être ajustée par tout moyen connu, le sel contenu dans le réservoir est immergé à l'issue du remplissage de la canalisation secondaire. Une fois ce remplissage effectué, la pompe 36 provoque la circulation du liquide dans la boucle en circuit fermé à un débit choisi pourqu'il devienne rapidement saturé.

Le fonctionnement en régime établi de ce dispositif ne diffère pas de celui du dispositif décrit en relation avec la figure 3.

La présente invention n'est pas limitée aux exemples de réalisation qui viennent d'être décrits et elle est susceptible de variantes. En particulier, dans les circuits de préparation de solution comprenant plusieurs canalisations secondaires, ces canalisations secondaires peuvent être reliées en série à la canalisation principale et non pas seulement en parallèle comme dans le mode de réalisation représenté sur la figure 1.

## Revendications

1. Dispositif de préparation d'une solution à usage médical à partir d'un liquide et d'au moins un sel pulvérulent ou cristallisé, comprenant:
- une canalisation principale (11) ayant une première extrémité reliée à une source de liquide (10) et une seconde extrémité pour délivrer la solution ;
- au moins une canalisation secondaire (15, 19 ; 16, 20) reliée en dérivation à la canalisation principale (11), cette canalisation secondaire comprenant un réservoir (13, 14) contenant un sel pulvérulent ou cristallisé et ayant un orifice d'entrée et un orifice de sortie situés de préférence respectivement à un point haut et à un point bas du réservoir (13, 14),
- des moyens (12, 21, 22, 36) pour faire circuler du liquide dans les canalisations comprenant
- des moyens de pompage (21, 22, 36) disposés en aval du réservoir (13, 14) sur la canalisation secondaire (15, 19 ; 16, 20),
ce dispositif étant caractérisé en _{,}ce qu'il comprend des moyens (17, 21 ; 18, 22) pour provoquer l'immersion du sel dans le réservoir en fermant initialement des moyens d'obturation (17, 18) disposés sur la canalisation secondaire (15, 19 ; 16, 20) en amont du réservoir (13, 14) en mettant en fonctionnement les moyens de pompage (21, 22, 36) et en ouvrant subséquemment les moyens d'obturation (17, 18).

2. Dispositif de préparation d'une solution à usage médical à partir d'un liquide et d'au moins un sel pulvérulent ou cristallisé, comprenant :
- une canalisation principale (11) ayant une première extrémité reliée à une source de liquide (10) et une seconde extrémité pour délivrer la solution ;
- au moins une canalisation secondaire (16, 20) reliée en dérivation à la canalisation principale (11), cette canalisation secondaire comprenant un réservoir (14) contenant un sel pulvérulent ou cristallisé et ayant un orifice d'entrée et un orifice de sortie situés de préférence respectivement à un point haut et à un point bas du réservoir (13, 14),
- des moyens (12, 36) pour faire circuler du liquide dans les canalisations,
ce dispositif étant caractérisé en ce qu'il comprend des moyens, (32, 12 ; 28, 36) pour provoquer l'immersion du sel dans le réservoir comprenant des moyens de mise en pression (32, 12 ; 28, 36) du réservoir (14).

3. Dispositif selon la revendication 2, caractérisé en ce que les moyens de mise en pression du réservoir (14) comprennent une pompe (12) et un organe d'étranglement (32) disposés sur la canalisation principale (11) respectivement en amont et en aval du point de jonction (30) de la canalisation principale (11) à la portion de canalisation secondaire (16) qui est reliée à l'orifice d'entrée du réservoir (14).

4. Dispositif selon la revendications 3, caractérisé en ce que le point de jonction (30) de la canalisation principale (11) à la portion de canalisation secondaire (16) qui est reliée à l'orifice d'entrée du réservoir (14) est situé en aval, par rapport au sens de circulation du liquide dans la canalisation principale (11), du point de jonction (24) de a canalisation principale (11) à la portion de canalisation secondaire (20) qui est reliée à l'orifice de sortie du réservoir (14).

5. Dispositif selon une des revendications 1 à 4, caractérisé en ce que la canalisation secondaire (15, 19 ; 16, 20) comporte une boucle comprenant le réservoir (14) et des moyens (36, 22a) pour faire circuler du liquide dans la boucle en circuit fermé.

6. Dispositif selon une des revendications 1 à 5, caractérisé en ce qu'il comporte plus d'une canalisation secondaire (15, 19 ; 16, 20) et en ce que les canalisations secondaires sont reliées en parallèle à la canalisation principale (11).

7. Dispositif selon une des revendications 1 à 5, caractérisé en ce qu'ils comporte plus d'une canalisation secondaire (15, 19 ; 16, 20) et en ce que les canalisations secondaires sont reliées en série à la canalisation principale (11).

8. Dispositif selon une des revendications 1 à 7, caractérisé en ce que le liquide de la source de liquide (10) est une solution.

## Claims

1. A device for preparing a solution for medical use from a liquid and at least one powdered or crystallized salt, comprising:
- a main line (11) having a first end connected to a source (10) of liquid and a second end for delivering the solution;
- at least one secondary line (15, 19; 16, 20) connected as a by-pass to the main line (11), this secondary line comprising a reservoir (13, 14) containing a powdered or crystallized salt and having an inlet opening and an outlet opening situated preferably at a high point and a low point of the reservoir (13, 14), respectively;
- means (12, 21, 22, 36) for circulating a liquid in the lines comprising pumping means (21, 22, 36) disposed in the secondary line (15, 19; 16, 20) downstream of the reservoir (13, 14);
this device being characterized in that it comprises means (17, 21; 18, 22) for causing the immersion of the salt in the reservoir by initially closing occluding means (17, 18) disposed in the secondary line (15, 19; 16, 20) upstream of the reservoir (13, 14), and in actuating the pumping means (21, 22, 36) and in subsequently opening the occluding means (17, 18).

2. A device for preparing a solution for medical use from a liquid and at least one powdered or crystallized salt, comprising:
- a main line (11) having a first end connected to a source (10) of liquid and a second end for delivering the solution;
- at least one secondary line (16, 20) connected as a by-pass to the main line (11), this secondary line comprising a reservoir (14) containing a powdered or crystallized salt and having an inlet opening and an outlet opening situated preferably at a high point and a low point of the reservoir (13, 14), respectively;
- means (12, 36) for circulating a liquid in the lines;
this device being characterized in that it comprises means (32, 12; 28, 36) for causing the immersion of salt in the reservoir including means (32, 12; 28, 36) for pressurizing the reservoir (14).

3. A device according to claim 2, characterized in that the means for pressurizing the reservoir (14) comprises a pump (12) and a constricting element (32) disposed in the main line (11) respectively upstream and downstream of the point of connection (30) of the main line (11) to the section of the secondary line (16) which is connected to the inlet opening of the reservoir (14).

4. A device according to claim 3, characterized in that the connection point (30) of the main line (11) to the section of the secondary line (16) which is connected to the inlet opening of the reservoir (14) is disposed downstream, with respect to the direction of circulation of the liquid in the main line (11), of the connection point (24) of the main line (11) to the section of the secondary line (20) which is connected to the outlet opening of the reservoir (14).

5. A device according to one of claims 1 to 4, characterized in that the secondary line (15, 19; 16, 20) includes a loop comprising the reservoir (14) and means (36, 22a) for causing the liquid to circulate in the loop in a closed circuit.

6. A device according to one of claims 1 to 5, characterized in that it comprises more than one secondary line (15, 19; 16, 20) and the secondary lines are connected in parallel to the main line (11).

7. A device according to one of claims 1 to 5 , characterized in that it comprises more than one secondary line (15, 19; 16, 20) and the secondary lines are connected in series to the main line (11).

8. A device according to one of claims 1 to 7, characterized in that the liquid of the liquid source (10) is a solution.

## Patentansprüche

1. Vorrichtung zur Herstellung einer Lösung für medizinischen Gebrauch aus einer Flüssigkeit und wenigstens einem pulverförmigen oder kristallisierten Salz, die folgendes aufweist:
- eine Hauptleitung (11) mit einem mit einer Flüssigkeitsquelle (10) verbundenen ersten Ende und einem zweiten Ende zum Liefern der Lösung,
- wenigstens eine von der Hauptleitung (11) abgezweigte Nebenleitung (15, 19; 16, 20) mit einem Vorratsbehälter (13, 14), der ein pulverförmiges oder kristallisiertes Salz enthält und eine Einlaßöffnung sowie eine Auslaßöffnung aufweist, die vorzugsweise an einer hoch bzw. tief gelegenen Stelle des Vorratsbehälters (13, 14) angeordnet sind,
- eine Einrichtung (12, 21, 22, 36) zum Umwälzen der Flüssigkeit in den Leitungen, die Pumpeinrichtungen (21, 22, 36) umfassen, welche stromab des Vorratsbehälters (13, 14) an der Nebenleitung (15, 19; 16, 20) angeordnet sind,
wobei diese Vorrichtung dadurch gekennzeichnet ist, daß sie eine Einrichtung (17, 21; 18, 22) zum Bewirken des Eintauchens des Salzes in den Vorratsbehälter durch anfängliches Schließen von Verschlußmitteln (17, 18), die stromauf des Vorratsbehälters (13, 14) an der Nebenleitung (15, 19 ; 16, 20) angeordnet sind, und durch Inbetriebsetzen der Pumpeinrichtungen (21, 22, 36) und durch anschließendes Öffnen der Verschlußmittel (17, 18) aufweist.

2. Vorrichtung zur Herstellung einer Lösung für medizinischen Gebrauch aus einer Flüssigkeit und wenigstens einem pulverförmigen oder kristallisierten Salz, die folgendes aufweist:
- eine Hauptleitung (11) mit einem mit einer Flüssigkeitsquelle (10) verbundenen ersten Ende und einem zweiten Ende zum Liefern der Lösung,
- wenigstens eine von der Hauptleitung (11) abgezweigte Nebenleitung (16, 20) mit einem Vorratsbehälter (14), der ein pulverförmiges oder kristallisiertes Salz enthält und eine Einlaßöffnung und eine Auslaßöffnung aufweist, die vorzugsweise an einer hoch bzw. tief gelegenen Stelle des Vorratsbehälters (13, 14) angeordnetsind,
- eine Einrichtung (12, 36) zum Umwälzen der Flüssigkeit in den Leitungen,
wobei diese Vorrichtung dadurch gekennzeichnet ist, daß sie Einrichtungen (32, 12; 28, 36) zum Bewirken des Eintauchens des Salzes im Vorratsbehälter aufweist, die Einrichtungen (32, 12; 28, 36) zum Unterdrucksetzen des Vorratsbehälters (14) umfassen.

3. Vorrichtung nach Anspruch 2, dadurch gekennzeichnet, daß die Einrichtungen zum Unterdrucksetzen des Vorratsbehälters (14) eine Pumpe (12) und ein Drosselorgan (32) umfassen, die an der Hauptleitung (11) stromauf bzw. stromab der Verbindungsstelle (30) der Hauptleitung (11) mit dem mit der Einlaßöffnung des Vorratsbehälters (14) verbundenen Teil der Nebenleitung (16) angeordnet ist.

4. Vorrichtung nach Anspruch 3, dadurch gekennzeichnet, daß die Verbindungsstelle (30) der Hauptleitung (11) mit dem Teil der Nebenleitung (16), der mit der Einlaßöffnung des Vorratsbehälters (14) verbundenen ist, bezüglich der Umwälzeinrichtung der Flüssigkeit in der Hauptleitung (11) stromab der Verbindungsstelle (24) der Hauptleitung (11) mit dem Teil der Nebenleitung (20), der mit der Auslaßöffnung des Vorratsbehälters (14) verbunden ist, angeordnet ist.

5. Vorrichtung nach einem der Ansprüche 1 bis 4, dadurch gekennzeichnet, daß die Nebenleitung (15, 19; 16, 20) eine den Vorratsbehälter (14) enthaltende Leitungsschleife und Einrichtungen (36, 22a) zum Umwälzen der Flüssigkeit in der Leitungsschleife als geschlossener Kreislauf enthält.

6. Vorrichtung nach einem der Ansprüche 1 bis 5, dadurch gekennzeichnet, daß sie mehr als eine Nebenleitung (15, 19; 16, 20) aufweist und daß die Nebenleitungen parallel mit der Hauptleitung (11) verbunden sind.

7. Vorrichtung nach einem der Ansprüche 1 bis 5, dadurch gekennzeichnet, daß sie mehr als eine Nebenleitung (15, 19; 16, 20) aufweist und daß die Nebenleitungen in Reihe mit der Hauptleitung (11) verbunden sind.

8. Vorrichtung nach einem der Ansprüche 1 bis 7, dadurch gekennzeichnet, daß es sich bei der Flüssigkeit aus der Flüssigkeitsquelle (10) um eine Lösung handelt.
